# EUROPÄISCHE PATENTSCHRIFT

(11) **EP 3 923 803 B1**
(45) Veröffentlichungstag und Bekanntmachung des Hinweises auf die Patenterteilung: **08.04.2026**
(21) Anmeldenummer: 20705181.4
(22) Anmeldetag: 13.02.2020
(51) Int. Cl.: A61B 5/11, G01L 1/00

(54) **SENSORELEMENT ZUR ERFASSUNG VON DEHNUNGEN BEI DER BEWEGUNG EINES KÖRPERTEILS EINES LEBEWESENS**
SENSOR ELEMENT FOR SENSING STRETCHING DURING A MOVEMENT OF A BODY PART OF A LIVING BEING
ÉLÉMENT CAPTEUR DESTINÉ À DÉTECTER DES EXTENSIONS LORS DU MOUVEMENT D'UNE PARTIE DU CORPS D'UN ÊTRE VIVANT

(30) Priorität: 14.02.2019 DE 102019201954
(43) Veröffentlichungstag der Anmeldung: 22.12.2021
(73) Patentinhaber: Fraunhofer-Gesellschaft zur Förderung der angewandten Forschung e.V., 80686 München (DE)
(72) Erfinder: MÄDER, Thomas, 09126 Chemnitz (DE); SENF, Björn, 09126 Chemnitz (DE); EBRECHT, Florian, 09111 Chemnitz (DE); SICHTING, Freddy, 09112 Chemnitz (DE)
(74) Vertreter: Pfenning, Meinig & Partner mbB
(86) Internationale Anmeldenummer: PCT/EP2020/053686
(87) Internationale Veröffentlichungsnummer: WO 2020/165306

(56) Entgegenhaltungen:
- WO-A1-2017/020111
- WO-A2-00/17615
- WO-A2-03/012384
- WO-A2-2016/168117
- US-A1- 2012 316 798
- US-A1- 2013 091 954
- US-A1- 2017 164 876

## Beschreibung

Die Erfindung betrifft ein Sensorelement nach Anspruch 1, das zur Erfassung von Dehnungen bei der Bewegung eines Körperteils eines Lebewesens dient, also für biomechanische Anwendungen.

Die Erfassung der Ausdehnung/Kontraktion an der Körperoberfläche von Lebewesen, insbesondere von Menschen und anderen Säugetieren, ist eine aktuelle Problemstellung. Anhand dieser Dehnung kann auf die Bewegung von Gliedmaßen, den Zustand von Gelenken und die Aktivität von Muskeln sowie auf die Dehnung von Bändern und Faszien geschlossen werden. So ist es denkbar Bewegungsabläufe an unterschiedlichen Körperpartien zu erfassen oder bspw. die Aktivität der Atmung durch die Aufweitung des Brustkorbs zu überwachen. Technologien zur Erfassung dieser Körperelement- bzw. Hautdehnung bieten eine Reihe von interessanten Anwendungen mit hohem Vermarktungspotenzial. Beispiele dafür sind:
- Wearables,
- Ansteuerung von Spiele-(Apps),
- Feedback für Therapie (Anfang/Verlauf/Ende),
- Mensch-Maschine-Interaktion / MRK,
- Fitness-Monitor,
- Kontrolle der technischen Ausführung von Sportübungen,
- Erforschung/Entwicklungen zum Bewegungsapparat,
- Aufzeichnung der Respiration im Alltag,
- Bewertung des Therapieerfolgs bei der Behandlung von Narben durch Elastizitätsmessungen,
- Sensorpflaster, Sensor-Einlegesohlen, Sensor-Handschuhe,
- Life Science-Produkte.

Für die Dehnungsmessung am menschlichen Körper eignen sich prinzipell optische Messsysteme oder applizierbare Dehnungssensoren. Zu letztgenannten zählen insbesondere resistive und kapazitive Sensoren. Deren Vorteile liegen in der Nutzbarkeit einfacher dazu einsetzbarer Auswertungselektronik mit einer hohen Flexibilität und Dehnbarkeit. Unter diesen beiden Sensorklassen gibt es wiederum vielfältige technologische Lösungen mit unterschiedlichen Eigenschaften. Beschleunigungs- und Lagesensoren werden ebenfalls für den Zweck der Erfassung von Körperbewegungen eingesetzt.

Die optischen Messsysteme sind vornehmlich stationär einsetzbar. Sie erfordern zudem die direkte Sichtbarkeit von Markern, die auf der Haut appliziert sind. Alltagssituationen oder mobile Messungen sind daher mit den optischen Messsystemen nicht realisierbar.

Bei der Nutzung von Beschleunigungssensoren ist ein regelmäßiges Kalibrieren erforderlich, was ein Anhalten der Bewegung erfordert. Diese Unterbrechungen verhindern natürliche Bewegungsabläufe und machen ein lückenloses Erfassen von Bewegungen unmöglich.

Einfache Dehnungsmessstreifen (DMS) und Sticksensoren, als resistive Sensoren auf Basis von Konstantan oder Pt/W-Drähten sind nur für geringe Dehnungen geeignet. Nachteilig an diesen Sensoren ist deren geringe Streckgrenze. Wegen ihrer geringen elastischen Dehnbarkeit sind sie nicht für wiederholte Dehnungen mit Amplituden größer als 0,5 % Dehnung einsetzbar. Nach wenigen Zyklen versagen diese Sensoren bei großen Amplituden. Für den Einsatz auf der Haut sind DMS daher nur an wenigen und ausgewählten Stellen geeignet.

Resistive Dehnungssensoren mit hoher Elastizität auf Basis von Nanomaterialien bestehen zumeist aus einem Dünnfilm, der mit nanoskaligen Strukturen gebildet ist, in Kombination mit einem flexiblen Substratmaterial. Änderungen der Mikrostruktur führen bei Dehnung zu einer Änderung des elektrischen Widerstands. Dabei überlagern sich häufig mehrere innere Phänomene, die die elektrische Widerstandsänderung bedingen. Wesentlich sind hier die Änderung der geometrischen Verhältnisse, die Ausbreitung von Rissen in den Dünnfilmen, die Änderung der elektrischen Übergangswiderstände zwischen Mikroelementen sowie Tunnel-Effekte. Solche resistiven Sensoren weisen zumeist eine vergleichsweise höhere Dehnbarkeit mit höherer Sensitivität auf, zeigen aber nichtlineares Verhalten und sind Hysterese-behaftet.

Kapazitive Sensoren bestehen aus einer hoch nachgiebigen dielektrischen Schicht im Sandwich zwischen zwei dehnbaren Elektroden. Die Änderung des Abstands der beiden Elektroden aufgrund von Dehnung bewirkt eine Änderung der elektrischen Kapazität als messbare Größe. Diese Klasse elastischer Dehnungssensoren zeigt eine hervorragende Dehnbarkeit, Linearität und ausgezeichnete Hystereseeigenschaften. Allerdings ist deren Sensitivität sehr gering.

Als leitfähiges Mikro- oder Nanomaterial für elastische Dehnungssensoren kommen CNT-, Rußpartikel-, Graphen-, Silbernanodraht- und Nanopartikel-Netzwerke zum Einsatz. Für das dielektrische Trägermaterial kommen vorwiegend Silikon-basierte Elastomere, Gummi sowie thermoplastische Elastomere zur Anwendung. Die Temperaturempfindlichkeit für Kohlenstoff-basierte Netzwerke sowie für diese Netzwerke aus Nano- und Mikropartikeln im Allgemeinen ist recht groß.

Nachteilig bei den angeführten Ansätzen ist die häufig auftretende starke Kopplung des Sensorsignals bei Dehnung und bei aufgebrachtem Druck. Druck wird bspw. durch anliegende bzw. gespannte Kleidung oder durch äußere Berührungen in den Sensor eingebracht. Dehnungs- und Druckwirkung sind aufgrund der Kopplung nicht unterscheidbar. Bisher existieren auch nur ungenügende Lösungen zur Aufbau- und Verbindungstechnik dieser Sensoren. Es wurde bisher kein kompaktes Sensorsystem aus Sensor, Energiespeicher, Elektronik zur Messdatenverarbeitung und -kommunikation, das robust und zuverlässig eine Langzeitüberwachung erlaubt, umgesetzt. Eine ausgereifte und marktfähige technische Lösung sollte daher diese Aspekte mit einbeziehen.

Kupfer-basierte Leiter haben eine geringe Streckgrenze, verfestigen sich durch Verformung und brechen nach einer gewissen Zahl von wechselseitigen Verformungen.

So sind aus WO 03/012384 A2 ein Verfahren und eine Vorrichtung zur Messung von Belastungen mit Formgedächtnislegierungen bekannt.

WO 2017/020111 A1 betrifft Bekleidung mit Zugsensoren.

Ein System zur Bewertung und Anzeige der Bewegung eines Sportlers geht aus US 2012/0316798 A1 hervor.

Es ist daher Aufgabe der Erfindung, Möglichkeiten für eine dauerhafte Erfassung von Dehnungen, die bei Bewegung von Körperteilen eines Lebewesens auftreten, anzugeben und dabei relativ große Dehnungen von mehr als 5 %, bevorzugt mehr als 8 % mit ausreichender Messgenauigkeit zu berücksichtigen sowie das jeweilige Lebewesen, wenn überhaupt nur geringfügig während der zu beachtenden Bewegungsabläufe zu beeinflussen.

Erfindungsgemäß wird diese Aufgabe mit einem Sensorelement, das die Merkmale des Anspruchs 1 aufweist, gelöst. Vorteilhafte Ausgestaltungen und Weiterbildungen der Erfindung können mit in untergeordneten Ansprüchen bezeichneten Merkmalen realisiert werden.

Das erfindungsgemäße Sensorelement ist am jeweiligen Körperteil befestigbar. Dabei ist das Sensorelement mit mindestens einem elektrischen draht- oder streifenförmigen elektrischen Leiter gebildet, der an eine elektrische Spannungsquelle und mit einer Einrichtung, die zur Bestimmung des durch den Leiter fließenden elektrischen Stroms, der elektrischen Spannung und/oder des elektrischen Widerstands ausgebildet ist, anschließbar ist. Der elektrische Leiter ist aus einem Formgedächtnismetall gebildet, das insbesondere eine Formgedächtnislegierung ist und nachfolgend als FGL bezeichnet werden soll. Der elektrische Leiter ist mit einem elastisch verformbaren Trägerelement verbunden.

Der elektrische Leiter kann mit einer elektrisch isolierenden Beschichtung an seiner Oberfläche versehen, und dabei mittels der Beschichtung haftend an der Haut des jeweiligen Körperteils befestigbar sein. So kann der mindestens eine elektrische Leiter an einer Hautoberfläche eines jeweiligen Körperteils appliziert und durch die Haftwirkung dort gehalten werden. Eine Beeinflussung durch Feuchtigkeit auf der Hautoberfläche kann mittels der Beschichtung vermieden werden. Das Beschichtungsmaterial kann so gewählt werden, dass ein manuelles Ablösen und ggf. eine Wiederverwendung eines solchen Sensorelements möglich ist. Die Haftwirkung kann durch adhäsives Verhalten des Beschichtungswerkstoffs und/oder durch eine geeignete Oberflächenstrukturierung der Beschichtung, beispielsweise mittels Saugnäpfen oder einer Gecko-Struktur erreicht werden.

Der mindestens eine elektrische Leiter kann unter einer Hautoberfläche implantierbar sein. Dabei sollten an den Stirnenden des elektrischen Leiters vorhandene oder dort anschließbare elektrische Kontaktelemente zumindest bis an die Hautoberfläche reichen bzw. bis dorthin geführt sein. Es ist aber auch eine induktive Übertragung elektrischer Energie und von mit dem Sensorelement erfassten Dehnungsmesswerten möglich. Dazu kann man eine entsprechende Antenne oder einen Dipol ebenfalls implantieren und für die Energie- und Datenübertragung zusätzlich eine externe Antenne einsetzen.

Der elektrische Leiter kann sinusförmig, mäanderförmig, in Zick-Zack-Form, in Form eines Halbkreismusters oder in Kombination dieser Formen gebogen am jeweiligen Körperteil befestigbar sein. Die gebogene Form kann durch eine thermische Behandlung mit gezielter Verformung erhalten werden. Es ist aber auch ein entsprechendes Heraustrennen aus beispielsweise einer Platte in der gewünschten gebogenen Form möglich.

Eine pseudoelastische FGL kann dauerhaft als Leiter fungieren. Zudem bietet sich die Möglichkeit einer zusätzlichen Ordnungsfunktion. Durch Wärmebehandlung lässt sich in das FGL eine Form einprägen. Bei Entlastung ist das pseudoelastische Material immer bestrebt in diese Form zurück zu gelangen. Ein elektrischer Leiter könnte sich auf diese Art selbst einrollen.

Der elektrische Leiter sollte vorteilhaft so gebogen sein, dass seine zwei Stirnenden für den Anschluss an elektrische Kontaktelemente in einem Abstand zu- und unmittelbar nebeneinander angeordnet sind. Dadurch kann eine einfache und unkomplizierte elektrische Kontaktierung zu der elektrischen Spannungsquelle und der Einrichtung, die zur Bestimmung des durch den Leiter fließenden elektrischen Stroms, der elektrischen Spannung und/oder des elektrischen Widerstands ausgebildet ist, ermöglicht werden.

Das Trägerelement kann aus einem elastisch verformbaren Material, das mit dem elektrischen Leiter stoffschlüssig verbunden oder in das der elektrische Leiter zumindest teilweise eingebettet ist, gebildet sein. Es kann aber auch mit einem textilen Gebilde gebildet sein, mit dem der elektrische Leiter z.B. formschlüssig verbunden ist. Der elektrische Leiter kann in das textile Gebilde beispielsweise eingewebt oder mittels einer Stickverbindung verbunden sein. Vorteilhaft ist ein textiles Gebilde mit elastisch verformbaren Fasern gebildet. Deren Elastizität kann durch das Fasermaterial, die Art der Faser, die Anzahl der Fasern und die Festigkeit des textilen Verbundes beeinflusst werden.

Ein elastisch verformbares Material, mit dem ein Trägerelement gebildet ist, kann bei Anwendung der Erfindung zusätzlich ggf. anisotrope mechanische Eigenschaften aufweisen. Dazu kann ein elastisch verformbares Material eine Dehnung in eine Längsrichtung bei minimaler Querkontraktion ausführen. Die Dehnung in Längsrichtung kann eine bevorzugte Messrichtung eines Sensorelements sein.

Das Trägerelement kann in einer vorteilhaften Ausgestaltung in Form eines Gurtes oder eines Ringes ausgebildet sein, so dass es ein Körperteil, bei einem Tragen an einem Lebewesen, vollumfänglich umschließen kann. So kann ein Sensorelement einfach durch Überstreifen an einem Körperteil befestigt und zur Erfassung von Dehnungen genutzt werden. Dies ist beispielsweise an der Stirn eines Kopfes, der Brust, den Armen oder auch den Beinen problemlos möglich. Die jeweilige Länge kann durch einen entsprechenden Gurtverschluss oder Ringdurchmesser bzw. -umfang an das jeweilige Körperteil individuell angepasst werden. Ein Trägerelement kann aber auch in Form eines Kleidungsstückes, beispielsweise einem Strumpf oder Handschuh ausgebildet sein.

Das Trägerelement ist als ein die maximale Dehnung des elektrischen Leiters limitierendes Element ausgebildet. Dadurch kann eine Beschädigung des elektrischen Leiters bei zu großer Dehnung vermieden werden. Dafür sind in einem Trägerelement entsprechende Verstärkungselemente, die den maximal möglichen Weg in der jeweiligen Achsrichtung bei der Bewegung des Körperteils auftretenden Dehnung begrenzen, vorhanden. Die Verstärkungselemente werden dabei auf Zugkraft beansprucht und Beenden eine weitere Dehnung, wenn der maximal zulässige Weg bei der Verformung des elektrischen Leiters erreicht worden ist. Die Verstärkungselemente bilden also eine Art Ankerelement. Sie können entsprechend an einem elastisch verformbaren aus Polymer bestehenden Trägerelement vorhanden, daran befestigt oder im Polymer eingebettet sein. Bei einem Trägerelement, das mit einem textilen Gebilde gebildet ist, können entsprechende Fasern bzw. Fäden mit eingewebt oder am textilen Gebilde befestigt sein. Solche Fasern bzw. Fäden sollten eine größere Festigkeit als die die bei der Herstellung des textilen Gebildes eingesetzt worden sind, aufweisen. Sie können beispielsweise Metallfasern sein, wobei aber die elektrische Isolation zu dem elektrischen Leiter beachtet werden sollte.

Ein elastisches Trägerelement kann form- und/oder stoffschlüssige Verbindungselemente aufweisen, die reversibel auf die Oberfläche von konfektionierten textilen Flächengebilden appliziert und wieder gelöst werden können.

Der elektrische Leiter kann vorteilhaft so an einem Körperteil befestigbar sein, dass seine maximale Messempfindlichkeit in einer Achsrichtung ausgerichtet ist, die mindestens 10 ° und maximal 80 ° von der zu bestimmenden Dehnungsrichtung des jeweiligen Körperteils bei einer zu beachtenden Bewegung abweicht. Dadurch kann ein Verstärkungseffekt erreicht werden, indem größere Dehnungen berücksichtigt werden, als dies der elektrische Leiter eigentlich zulassen würde, da er nicht maximal gedehnt werden muss, als dies die eigentliche Dehnungsamplitude erfordern würde. Dadurch können Beschädigungen am elektrischen Leiter vermieden und die Lebensdauer erhöht werden. So können auch Dehnungen > 10 % berücksichtigt werden, ohne dass es zu Problemen kommt. Der Winkel sollte bevorzugt zwischen 10 ° und 30 ° von der eigentlichen Dehnungsrichtung bei der zu überwachenden Bewegung auftritt, abweichen, um eine gute Messempfindlichkeit und Bruchsicherheit des elektrischen Leiters zu gewährleisten.

Es ist eine Adaption des Sensorelements an unterschiedliche Körperpartien bzw. Körperteilen mit variierender Maximaldehnung realisierbar. Die elastische Trägerstruktur kann einerseits selbstklebend zur direkten Fixierung auf der Haut ausgeführt oder in Form eines Kleidungsstücks bzw. einer umspannenden Gestalt ausgeführt sein. Die umspannende Gestalt kann sich nach dem Aufziehen über Körperpartien (Torso, Arme, Beine, Füße, Hände) aufgrund ihrer Eigenelastizität selbst halten.

Eine versetzte bzw. gespiegelte Anordnung der Muster eines entsprechend gebogenen elektrischen Leiters kann zur Hin- und Rückführung des länglichen FGL-Materials und Realisierung zweier dicht nebeneinander liegender Kontaktstellen genutzt werden.

Es ist eine Fixierung und lokale Versteifung in einem oder mehreren Umkehrpunkten des länglichen FGL-Materials im Verlauf des Musters, an denen ein Richtungswechsel eines elektrischen Leiters erfolgt, möglich.

Es können auch Querversteifung am elastischen Trägerelement zur Reduktion der Querkontraktion bzw. dem Nutzen einer erhöhten Breite der beidseitig von der Sensorstruktur verlaufenden Haut-Klebefläche dieser Trägerstruktur eingesetzt werden.

Insbesondere die Realisierung einer Temperaturkompensation ist durch Ausbildung und elektrische Verschaltung mehrerer elektrischer Leiter an einem Sensorelement als Halb-/Vollbrücke möglich, wobei die elektrischen Leiter in verschiedenen Orientierungswinkeln zueinander ausgerichtet sein sollten.

An einem Sensorelement können auch mehrere elektrische Leiter vorhanden sein, die unterschiedliche Bewegungen auch unabhängig voneinander überwachen können.

Ein textiles Gebilde als Trägerelement kann auch mit reversibel haftendem Material an der Oberfläche (Klettverschluss, Pilzverschluss, Dauerklebeband etc.) zum wiederholten Anhaften auf der Haut oder auf textilen Strukturen (Bandagen, Orthesen, Kleidung, Verbände, Klettpads etc.) ausgebildet sein.

Ein Sensorelement kann auch als eigen-stabilisierter Sensor ausgebildet sein, so dass im Messverlauf keine Sensordrift auftreten kann. Dafür erfährt das verwendete FGL-Material eine geeignete thermo-mechanische Vorbehandlung bzw. ein Training. Infolgedessen vollzieht sich die Phasenumwandlung bei geringerer Kraft und das Auftreten plastischer Verformungsanteile ist minimiert.

Mit Nutzung eines erfindungsgemäßen Sensorelements zur Erfassung von Hautdehnungen, der Aufweitung des Brustkorbs infolge der Atmung, der Bewegung von Gelenken kann eine Kalibrierung der Hautdehnung auf definierte Gelenkbewegungen erfolgen.

Insbesondere die Einrichtung, die zur Bestimmung des durch den Leiter fließenden elektrischen Stroms, der elektrischen Spannung und/oder des elektrischen Widerstands ausgebildet ist, und bevorzugt zusätzlich auch die elektrische Spannungsquelle sollte als tragbare Miniatur-Elektronik zur Signalerfassung und drahtlosen Signalübertragung ausgebildet sein. Sie sollte wiederverwendet werden können. Sie kann/können ebenso durch Aufkleben oder Ankletten auf die Sensorträgerstruktur in Sensornähe fixiert werden und ein so geringes Eigengewicht sowie eine so kleine Bauform aufweisen, dabei mechanisch stabil bzw. flexibel sein, so dass natürliche Bewegungsabläufe ungehindert möglich sind und die Elektronik dabei keinen Schaden nimmt.

Die elektrische Aufbau- und Verbindungstechnik sollte ein einfaches und zuverlässiges Verbinden zwischen elektrischem Leiter und Elektronik sowie elektrischer Spannungsquelle gewährleisten können. Dies kann bspw. durch Stecken mit flachen Steckverbindern oder durch magnetische Fixierung von Kontaktanschlüssen (sensorseitig) zu Kontaktstiften (elektronikseitig) erreicht werden, wobei die sensorseitigen Steckverbindungsteile bzw. Kontaktpads stoffschlüssig mit dem länglich geformten FGL-Material, das den mindestens einen elektrischen Leiter bildet, verbunden sein sollten.

Die elektrische Isolierung der Oberfläche eines elektrischen Leiters, der Fügeverbindungen und aller nicht für den lösbaren Kontakt erforderlichen leitfähigen Bestandteile der sensorseitigen Steckverbindungsteile bzw. Kontaktanschlüsse kann durch eine geeignete Beschichtung erreicht werden.

So kann beispielsweise ein Kinesio-Tape als elastisches Trägermaterial mit 25 µm dickem Sensordraht verlegt im Zick-Zack-Muster und mittels Nähfäden fixiert ein Sensorelement bilden. Das Kinesio-Tape bildet das Trägerelement und der Sensordraht bildet den elektrischen Leiter.

Eine Silikon-Folie kann ebenfalls als elastisches Trägermaterial für ein Trägerelement mit 25 µm dickem Sensordraht als elektrischer Leiter, der im Sinus-Muster gebogen und mittels Kleben bzw. stoffschlüssiger Bindung im/am Silikon fixiert ist, eingesetzt werden.

Ein hautenges elastisches Oberteil-Kleidungsstück mit Sensordraht, als elektrischer Leiter, der quer zur Körper-Längsachse entlang der Brust orientiert bzw. in einem Übersetzungswinkel quer zur Körper-Längsachse wiederkehrend orientiert ausgerichtet ist, kann ebenfalls ein Trägerelement eines Sensorelements bilden, wodurch die Erfassung der Atmung eines Lebewesens möglich ist.

Es kann auch ein textiler Sensorstrumpf mit aufgebrachtem und textiltechnisch fixiertem Sensordraht als elektrischer Leiter, verlegt im Zick-Zack-Muster, ein Sensorelement bilden, mit dem insbesondere die Verformung des Fußes bei Bewegung erfasst werden kann.

Sensorstreifen aus textilem Trägermaterial mit Zick-Zack-Muster des gebogenen elektrischen Leiters können mit Klett-Oberfläche an einem Sensorelement mit diesem Trägermaterial, der sich auf die festgelegte Sensorposition einer Bandage, einer Orthese oder auf Klett-Klebepads, die zuvor auf die Haut aufgebracht worden, aufkletten lässt, für Sensorelemente eingesetzt werden.

Durch die neuartige Sensorstruktur und die damit möglichen biomechanischen Anwendungen werden im Wesentlichen Dehnungsmessungen mit hoher Dehnung (>10 %) und Zyklenzahl ermöglicht. Dehnungen der Körperoberfläche oder von Muskeln können hierdurch wiederholbar, zuverlässig und längerfristig erfasst werden. Die Dehnungsmessung ist zudem mit einfachen Mitteln und bei geringen Kosten möglich. Das Sensorelement ist klein (Format Streichholzschachtel bzw. kleiner) ausführbar.

Die besonderen Vorteile lauten:
- Erfassung von Dehnungen >10 %,
- Einstellbarkeit der maximal erreichbaren Dehnung durch variable Übersetzung,
- hohe Sensitivität im Vgl. zu konventionellen resisitiven Sensormaterialien (bspw. Konstantan),
- geringe Hysterese,
- hohe Linearität,
- geringe Druckempfindlichkeit,
- Langzeitstabilität der Messwerte.

Die genannten Wirkungen und Vorteile reduzieren den Aufwand und die Kosten für Dehnungsmessungen im Vergleich zum Stand der Technik.

Es können für elektrische Leiter übliche und an sich bekannte FGL eingesetzt werden.

Pseudoelastische FGL weisen eine große elastische Dehnbarkeitung von bis zu 15 % auf. Die große Dehnung wird dabei durch eine reversible Phasenumwandlung des Materials ermöglicht.

Mit der beschriebenen Phasenumwandlung ist zudem eine signifikante Änderung des elektrischen Widerstands verbunden. Diese kann zur Messung von Dehnungen genutzt werden. FGL können damit als Sensoren zur Überwachung der Dehnung eingesetzt werden. Es lassen sich große Dehnungen und davon abgeleitete Größen (z.B. Kräfte) erfassen. FGL-Sensoren können sowohl auf der Oberfläche appliziert als auch ins Innere einer Struktur integriert werden.

## Patentansprüche

1. Sensorelement zur Erfassung von Dehnungen bei der Bewegung eines Körperteil eines Lebewesens, das am jeweiligen Körperteil befestigbar ist, wobei
das Sensorelement mit mindestens einem elektrischen draht- oder streifenförmigen Leiter gebildet ist, der an eine elektrische Spannungsquelle und mit einer Einrichtung, die zur Bestimmung des durch den Leiter fließenden elektrischen Stroms, der elektrischen Spannung und/oder des elektrischen Widerstands ausgebildet ist, anschließbar ist, und
der elektrische Leiter aus einem Formgedächtnismetall gebildet ist und
mit einem elastisch verformbaren Trägerelement verbunden an der Oberfläche des jeweiligen Körperteils befestigbar ist,
**dadurch gekennzeichnet, dass**
das Trägerelement als ein die maximale Dehnung des elektrischen Leiters limitierendes Element ausgebildet ist, wobei am Trägerelement Verstärkungselemente, die den maximal möglichen Weg in der jeweiligen Achsrichtung der Bewegung eines Körperteils auftretende Dehnung begrenzen, vorhanden sind.

2. Sensorelement nach Anspruch 1, **dadurch gekennzeichnet, dass** der elektrische Leiter sinusförmig, mäanderförmig, in Zick-Zack-Form, in Form eines Halbkreismusters oder in der Kombination dieser Formen gebogen am jeweiligen Körperteil befestigbar ist.

3. Sensorelement nach einem der vorhergehenden Ansprüche, **dadurch gekennzeichnet, dass** der elektrische Leiter so gebogen ist, dass seine zwei Stirnenden für den Anschluss an elektrische Kontaktelemente in einem Abstand zu- und unmittelbar nebeneinander angeordnet sind.

4. Sensorelement nach einem der vorhergehenden Ansprüche, **dadurch gekennzeichnet, dass** das Trägerelement aus einem elastisch verformbaren Material, das
mit dem elektrischen Leiter stoffschlüssig verbunden oder in das der elektrische Leiter zumindest teilweise eingebettet ist, oder
das Trägerelement mit einem textilen Gebilde, mit dem der elektrische Leiter formschlüssig verbunden ist,
ausgestaltet ist.

5. Sensorelement nach einem der vorhergehenden Ansprüche, **dadurch gekennzeichnet, dass** das Trägerelement in Form eines Gurtes, eines Ringes oder Kleidungsstückes ausgebildet ist, so dass es ein Körperteil, bei einem Tragen an einem Lebewesen, vollumfänglich umschließt.

6. Sensorelement nach einem der vorhergehenden Ansprüche, **dadurch gekennzeichnet, dass** das elastische Trägerelement form- und/oder stoffschlüssige Verbindungselemente aufweist und reversibel auf die Oberfläche von konfektionierten textilen Flächengebilden applizierbar und lösbar ausgebildet ist.

## Claims

1. A sensor element for detecting stretching during the movement of a body part of a living being, which can be attached to the respective body part, wherein
the sensor element is formed with at least one wire or strip-shaped electrical conductor which can be connected to an electrical voltage source and to a device which is designed to determine the electrical current flowing through the conductor, the electrical voltage and/or the electrical resistance, and
the electrical conductor is formed from a shape-memory metal and
can be attached to the surface of the respective body part with an elastically deformable carrier element,
**characterized in that**
the carrier element is designed as an element limiting the maximum stretch of the electrical conductor, wherein reinforcing elements are disposed on the carrier element which limit the maximum possible elongation occurring in the respective axial direction of movement of a body part.

2. The sensor element according to claim 1, **characterized in that** the electrical conductor can be attached to the respective body part in a sinusoidal, meandering, zigzag or semicircular shape or in a combination of these shapes.

3. The sensor element according to one of the preceding claims, **characterized in that** the electrical conductor is bent in such a way that its two front ends are arranged at a distance from and directly adjacent to one another for connection to electrical contact elements.

4. The sensor element according to one of the preceding claims, **characterized in that** the carrier element is designed with an elastically deformable material
that is materially bonded to the electrical conductor or in which the electrical conductor is at least partially embedded, or
the carrier element is designed with a textile structure to which the electrical conductor is positively connected.

5. The sensor element according to one of the preceding claims, **characterized in that** the carrier element is designed in the form of a belt, a ring or an article of clothing, so that it fully encloses a part of the body when worn on a living being.

6. The sensor element according to one of the preceding claims, **characterized in that** the elastic carrier element has form-fitting and/or material-fitting connecting elements and can be reversibly applied to and detached from the surface of ready-made textile fabrics.

## Revendications

1. Elément capteur pour la détection d'extensions lors du mouvement d'une partie du corps d'un être vivant, qui peut être fixé à la partie du corps concernée, dans lequel
l'élément capteur est formé avec au moins un conducteur électrique en forme de fil ou en forme de bande, qui peut être raccordé à une source de tension électrique et avec un dispositif qui est réalisé pour déterminer le courant électrique, la tension électrique et/ou la résistance électrique circulant à travers le conducteur, et
le conducteur électrique est formé d'un métal à mémoire de forme et
peut être fixé à la surface de la partie du corps concernée en étant relié à un élément de support élastiquement déformable,
**caractérisé en ce que**
l'élément de support est réalisé comme un élément limitant l'allongement maximal du conducteur électrique, dans lequel des éléments de renforcement, qui délimitent le trajet maximal possible de l'allongement dans la direction axiale respective du mouvement d'une partie du corps, sont présents sur l'élément de support.

2. Élément capteur selon la revendication 1, **caractérisé en ce que** le conducteur électrique peut être fixé à la partie du corps concernée en étant plié de manière sinusoïdale, de manière sinueuse, en forme de zigzag, en forme de motif semi-circulaire ou dans la combinaison de ces formes.

3. Élément capteur selon l'une quelconque des revendications précédentes, **caractérisé en ce que** le conducteur électrique est plié de telle sorte que ses deux extrémités frontales sont disposées à distance l'une de l'autre et directement côte à côte pour le raccordement à des éléments de contact électriques.

4. Elément capteur selon l'une quelconque des revendications précédentes, **caractérisé en ce que** l'élément de support est conçu à partir d'un matériau élastiquement déformable, qui
est relié au conducteur électrique par liaison de matière ou dans lequel le conducteur électrique est au moins partiellement intégré, ou
l'élément de support est conçu avec une structure textile à laquelle le conducteur électrique est relié par complémentarité de formes.

5. Elément capteur selon l'une quelconque des revendications précédentes, **caractérisé en ce que** l'élément de support est réalisé sous la forme d'une ceinture, d'un anneau ou d'un vêtement, de sorte qu'il entoure entièrement une partie du corps, lorsqu'il est porté sur un être vivant.

6. Elément capteur selon l'une quelconque des revendications précédentes, **caractérisé en ce que** l'élément de support élastique présente des éléments de liaison par complémentarité de forme et/ou de matière et est réalisé de manière à pouvoir être appliqué de manière réversible et amovible sur la surface de produits textiles plats confectionnés.
